Europäisches Patentamt

(19) European Patent Office

Office européen des brevets

(11) Numéro de publication: **0 218 857**
B1

(12) **FASCICULE DE BREVET EUROPEEN**

(45) Date de publication du fascicule du brevet:
08.08.90

(51) Int. Cl.⁵: **A61F 13/15, B31D 1/04**

(21) Numéro de dépôt: 86111718.2

(22) Date de dépôt: 25.08.86

(54) Matelas absorbant en forme de sablier pour une couche-culotte et fabrication en continu de tels matelas.

(30) Priorité: 27.08.85 FR 8512807

(43) Date de publication de la demande:
22.04.87 Bulletin 87/17

(45) Mention de la délivrance du brevet:
08.08.90 Bulletin 90/32

(84) Etats contractants désignés:
AT BE CH DE GB IT LI NL

(56) Documents cités:
WO-A-83/03051

(73) Titulaire: PEAUDOUCE, 59, Rue de la Vignette,
F-59126 Linselles(FR)

(72) Inventeur: de Jonckheere, Raphael, 797 Domaine de la
Vigne, F-59910 Bondues(FR)
Inventeur: Rousseau, Jean Luc, 233, Rue Jean Jaurès,
F-59170 Croix(FR)

(74) Mandataire: Casalonga, Axel et al, BUREAU D.A.
CASALONGA - JOSSE Morassistrasse 8,
D-8000 München 5(DE)

## Description

La présente invention se rapporte à un matelas absorbant en forme de sablier, c'est-à-dire ayant une forme générale rectangulaire avec deux échancrures latérales opposées, pour une couche-culotte, ainsi qu'à un procédé de fabrication en continu de tels matelas.

Les matelas absorbants en forme de sablier comprennent, dans le sens de la longueur du matelas, deux zones d'extrémités et une zone intermédiaire d'entrejambe de largeur inférieure à celle des zones d'extrémités. Pour des raisons d'efficacité, il est désirable que ces matelas présentent, dans le sens de la largeur, au moins dans la zone d'entrejambe, une partie centrale d'épaisseur supérieure à celle des parties latérales.

On connaît deux modes de réalisation de matelas absorbants de ce type.

Suivant un premier mode de réalisation, un matelas est constitué d'un tronçon rectangulaire de bande de matériau absorbant, dans chacun des deux bords latéraux opposés duquel sont pratiquées deux incisions, la partie délimitée entre ces deux incisions étant rabattue en direction du bord latéral opposé, de manière que les deux rabats opposés forment une double épaisseur dans la zone d'entrejambe du matelas. Ce rabattement n'est pas sans présenter des difficultés à des cadences élevées de fabrication. Par ailleurs, ce rabattement doit se faire avec une précision élevée de manière que les bords libres des rabats opposés soient contigüs. En effet, dans le cas contraire, ces bords peuvent, soit être espacés l'un de l'autre, soit se chevaucher, ce qui altère la fonction du matelas absorbant ainsi que l'aspect de la couche-culotte.

Selon un autre mode de réalisation connu, un matelas absorbant est formé par superposition d'un tronçon rectangulaire de matériau absorbant, ayant une largeur inférieure ou égale à la largeur d'entrejambe du matelas absorbant complet, à un tronçon de matériau absorbant en forme de sablier. Pour la fabrication en continu de tels matelas, on découpe une bande de matériau absorbant suivant une ligne de découpe longitudinale rectiligne en une première bande partielle relativement étroite et en une seconde bande partielle plus large, on découpe ensuite dans cette bande partielle plus large des échancrures diamétralement opposées et on pose la bande partielle plus étroite sur la bande partielle plus large avant de découper la bande composite transversalement dans les zones comprises entre les échancrures successives. Le principal inconvénient de ce procédé réside dans les chutes dues aux découpes pratiquées dans la bande partielle plus large en vue de la réalisation des échancrures.

Il est par ailleurs connu par le document WO-A 83/03 051 de fabriquer des couches-culottes ou articles analogues comprenant, entre une feuille intérieure perméable aux liquides et un matelas absorbant, une barrière imperméable aux liquides, sauf dans la partie centrale du matelas absorbant. Suivant un mode de réalisation, cette barrière est constituée par deux bandes découpées dans un film synthétique imperméable. Pour la fabrication en continu de ces couches-culottes, il est proposé, suivant le document en question, de découper une feuille imperméable continue selon un tracé formé d'une succession de segments de droite de même longueur, parallèles à l'axe longitudinal de la feuille, ces segments de droite successifs étant situés en quinconce alternativement de part et d'autre dudit axe et ayant leurs extrémités reliées par des segments obliques coupant ledit axe. On décale une première des bande ainsi obtenues latéralement d'une distance légèrement inférieure à la distance séparant les segments situés de part et d'autre dudit axe et on fait parcourir à l'autre desdites bandes un chemin qui est plus long que celui de la première bande d'une longueur égale au pas de la découpe. Les deux bandes forment ainsi ensemble une barrière continue présentant par intervalles une "fenêtre" médiane perméable, cette barrière continue étant déposée sur les matelas absorbants et découpée par intervalles en barrières individuelles recouvrant chacune un matelas absorbant. Ce procédé, concerne donc la réalisation en continu d'une barrière imperméable disposée entre le matelas absorbant et la feuille intérieure perméable de couches-culottes et non pas la fabrication de matelas absorbants en forme de sablier ayant une épaisseur accrue dans la zone d'entrejambe.

La présente invention a pour objet un matelas absorbant en forme de sablier qui puisse être fabriqué d'une manière simple, à grande cadence, sans aucune chute, avec une épaisseur accrue dans la partie médiane du matelas, notamment dans la zone d'entrejambe.

L'invention a également pour objet un procédé de fabrication particulièrement simple et rapide de tels matelas absorbants.

Le matelas absorbant conforme à l'invention en forme de sablier, à deux échancrures latérales opposées, pour une couche-culotte, comprend, dans le sens de sa longueur, deux zones d'extrémités et une zone intermédiaire d'entrejambe de largeur inférieure à celle des zones d'extrémités. Le matelas est constitué de deux parties de matelas superposées ayant des formes telles que le matelas présente, dans le sens de la largeur, une partie médiane d'épaisseur supérieure à celle des parties latérales. Selon l'invention, le matelas est constitué de deux parties de matelas identiques ayant chacune la même longueur que le matelas et une largeur inférieure à la largeur du matelas dans les zones d'extrémités. L'un des bords longitudinaux de chaque partie de matelas est rectiligne et l'autre est muni d'une échancrure identique aux échancrures du matelas. Les deux parties de matelas sont superposées partiellement, en position inversée, sous la forme d'un matelas composite de manière que leurs bords rectilignes se chevauchent et que leurs bords munis d'une échancrure soient situés sur les côtés longitudinaux opposés du matelas composite.

Suivant un mode de réalisation de l'invention, les deux parties de matelas se chevauchent sur une largeur correspondant à la largeur de chaque partie de matelas à l'endroit de son échancrure. Dans ce cas, le matelas composite présente une double épaisseur sur toute la largeur de la zone d'entrejambe.

. Suivant un autre mode de réalisation de l'invention, les deux parties de matelas se chevauchent sur une largeur inférieure à la largeur de chaque partie de matelas à l'endroit de son échancrure. Dans ce cas, le matelas composite ne présente une double épaisseur que sur une partie de la largeur de la zone d'entrejambe, partie située au milieu de la largeur de la zone d'entrejambe.

Pour la fabrication en continu de tels matelas absorbants, à partir d'un matériau absorbant en forme de bande, on divise en continu la bande, dans le sens longitudinal, en deux bandes partielles suivant une ligne de découpe ondulée symétrique par rapport à la ligne longitudinale médiane de la bande. La période de la ligne de découpe ondulée correspond à la longueur d'un matelas absorbant à fabriquer. On décale l'une des bandes partielles ainsi obtenues par rapport à l'autre bande partielle transversalement sur l'autre bande partielle d'une valeur supérieure à la largeur minimale de chaque bande partielle, et longitudinalement d'une valeur égale à 1/2, 1 1/2, 2 1/2 périodes de la ligne de découpe ondulée, de manière à obtenir une bande composite dont les deux bords latéraux sont ondulés de façon symétrique et dont la partie médiane présente une épaisseur double de celle des parties latérales. Enfin, on coupe la bande composite, à des intervalles correspondant à une période de la ligne de découpe ondulée, transversalement dans les zones de largeur maximale de la bande composite.

Il est possible de diviser la bande par une ligne de découpe sinusoïdale ou par une ligne de découpe à ondes trapézoïdale formée d'une alternance de segments de droite longitudinaux de même longueur, situés transversalement à égale distance de part et d'autre de la ligne longitudinale médiane de la bande, et de segments transversaux inversés reliant lesdits segments de droite entre eux.

Suivant un mode de réalisation préféré, on décale l'une des bandes partielles transversalement et longitudinalement par rapport à l'autre bande partielle en lui faisant décrire une boucle autour d'un rouleau dont l'axe est situé dans un plan parallèle au plan de la bande, au-dessus ou en dessous de ce dernier, et est incliné par rapport à la direction transversale de la bande.

Il est possible de décaler l'une des bandes partielles transversalement par rapport à l'autre bande partielle d'une valeur inférieure, égale ou supérieure à la largeur maximale de la bande partielle.

En se référant aux dessins schématiques annexés, on va décrire ci-après, plus en détail, un mode de réalisation illustratif et non limitatif de l'objet de l'invention; sur les dessins :

la figure 1 est une vue en plan d'un matelas absorbant composite conforme à l'invention;
la figure 2 est une coupe suivant II-II de la figure 1;
la figure 3 représente un tronçon de bande de matériau absorbant, divisé suivant une ligne de découpe ondulée;
la figure 4 illustre le décalage transversal et longitudinal d'une des deux bandes partielles par rapport à l'autre, pour amener la bande de la figure 3 sous la forme d'une bande composite prête à être découpée transversalement sous la forme de matelas absorbants.

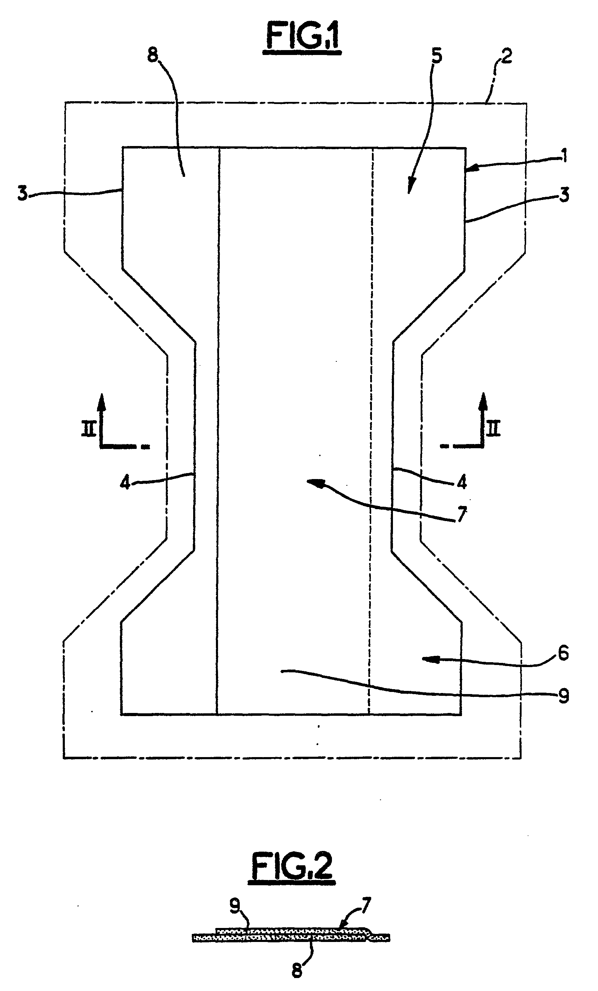

Selon les figures 1 et 2, un matelas absorbant 1 destiné à être incorporé à une couche-culotte 2 dont seul le contour est indiqué en traits mixtes sur la figure 1, présente, tout comme la couche-culotte 2, une forme en sablier, connue en soi. Cette forme en sablier est due à la présence, dans chacun des deux bords longitudinaux opposés 3 du matelas 1, d'une découpe 4 en forme d'échancrure trapézoïdale. Le matelas absorbant 1 comprend ainsi une partie d'extrémité 5 arrière, une partie d'extrémité 6 avant et une partie intermédiaire 7 d'entrejambe situées entre les deux échancrures 4. On reconnaît sur la figure 1 que la partie d'extrémité arrière 5 peut, de façon connue en soi, être quelque peu plus haute que la partie d'extrémité 6 avant. Pour la fermeture de la couche-culotte, la partie d'extrémité 5 arrière peut, de façon connue, être munie d'attaches adhésives, non représentées.

Le matelas absorbant 1 est composé de deux parties de matelas 8, 9 identiques. Chaque partie 8, 9 présente une forme générale rectangulaire, avec une échancrure 4 dans l'un des bords longitudinaux, alors que l'autre bord longitudinal est rectiligne. La largeur de chaque partie de matelas 8, 9, à l'endroit de l'échancrure 4, est inférieure à la largeur du matelas 1 dans la zone d'entrejambe 7. Les deux parties de matelas 8, 9 sont superposées partiellement, en position inversée, de manière que leurs bords longitudinaux rectilignes se chevauchent et que leurs bords longitudinaux munis d'une échancrure 4 soient situés sur les côtés opposés du matelas composite 1.

Ainsi, comme le montre surtout la figure 2, le matelas composite 1 présente, sur toute sa longueur, de part et d'autre d'une bande médiane à double épaisseur, d'une largeur inférieure à la largeur de la zone d'entrejambe 7, une bande d'épaisseur simple ayant une faible largeur dans la zone d'entrejambe 7 et une largeur accrue dans les deux zones d'extrémités 5 et 6.

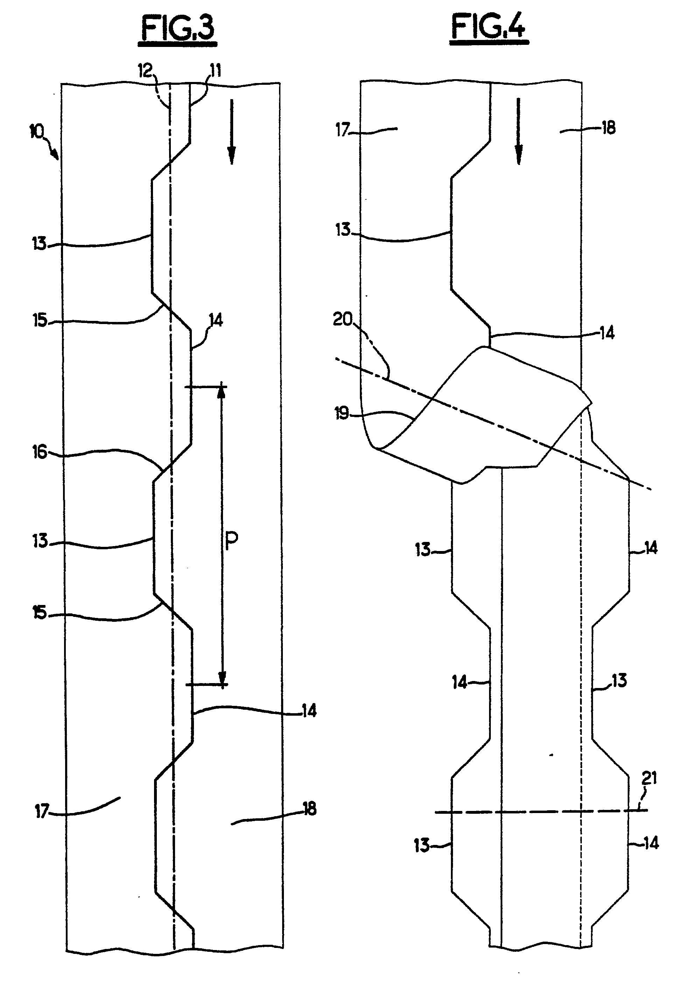

Pour fabriquer en continu des matelas absorbants suivant les figures 1 et 2, on divise, selon la figure 3, en continu, une bande 10 de matériau absorbant, en cours de défilement de la bande, dans le sens longitudinal de cette dernière, suivant une ligne de découpe ondulée 11 symétrique par rapport à la ligne longitudinale médiane 12 de la bande. La ligne de découpe ondulée 11 est une ligne à ondes trapézoïdales formée d'une alternance de segments de droite 13 et 14 longitudinaux, de même longueur, situés à égale distance de part et d'autre de la ligne médiane 12, et de segments de droite 15 et 16 obliques, inversés, reliant les segments de droite 13 et 14 entre eux. La période p de la ligne de découpe ondulée 11 est choisie de manière à correspondre à la longueur d'un matelas absorbant à fabriquer.

La bande 10 dont la largeur est supérieure à la largeur des matelas absorbants à fabriquer, est ainsi divisée sans chute par la ligne de découpe ondulée 11 en deux bandes partielles 17, 18 complémentaires, ayant chacune un bord longitudinal rectiligne et un bord longitudinal ondulé.

Selon la figure 4, on fait passer la bande partielle 17, alors que la bande partielle poursuit son trajet rectiligne, sous la forme d'une boucle 19 autour d'un rouleau non représenté dont l'axe 20 est contenu dans un plan parallèle au plan des bandes 17, 18, à distance au-dessus de ce plan. L'axe 20 est incliné par rapport à la longueur des bandes 17, 18, de telle manière que le passage en forme de boucle 19 de la bande 17 autour du rouleau provoque un décalage latéral de la bande 17 en direction de la bande 18, en même temps qu'un décalage longitudinal d'une période et demi par rapport à la bande 18. A la suite de la boucle 19, la bande partielle 17 se trouve donc en superposition partielle avec la bande 18, de telle manière que le bord longitudinal ondulé de chaque bande partielle 17, 18 dépasse latéralement le bord longitudinal rectiligne de l'autre bande partielle 18, 19. On obtient ainsi une bande composite ayant une double épaisseur dans sa partie médiane et, de part et d'autre de cette partie médiane de double épaisseur, des parties d'épaisseur simple à bords extérieurs ondulés de façon symétrique.

Pour former des matelas absorbants individuels à partir d'une telle bande composite, il suffit de découper la bande transversalement, au droit de chaque zone de largeur accrue, à des intervalles correspondant à la période p, comme indiqué en 21 sur la figure 4. On remarque que la ligne de découpe transversale 21 ne passe pas par le milieu des zones de largeur accrue de la bande composite, ce qui permet d'obtenir des matelas avec des zones d'extrémités de hauteurs différentes, comme représenté sur la figure 1.

Il va de soi que le mode de réalisation décrit ci-dessus et illustré par les dessins annexés, n'a été donné qu'à titre d'exemple non limitatif et que de nombreuses modifications et variantes sont possibles dans le cadre de l'invention.

Ainsi, la bande 10 pourrait également être découpée suivant une ligne ondulée sinusoïdale, auquel cas le matelas absorbant présenterait des bords longitudinaux curvilignes. Par ailleurs, le décalage longitudinal de l'une des bandes partielles par rapport à l'autre, par formation d'une boucle 19, pourrait également correspondre à une demi-période ou à deux périodes et demi, etc... Enfin, le décalage transversal de l'une des bandes partielles par rapport à l'autre, en vue de la superposition des deux bandes, pourrait également être moins important que selon la figure 4, auquel cas, toute la zone d'entrejambe pourrait être à double épaisseur, ou plus important, auquel cas, la largeur de chevauchement des deux parties de matelas serait plus faible.

Le matelas absorbant conforme à l'invention peut être fabriqué à partir de n'importe quel matériau absorbant en forme de bande, par exemple de la ouate de cellulose ("fluff") avec ou sans matériaux superabsorbants incorporés et avec ou sans matériaux de doublage tels que papier, etc...

**Revendications**

1. Matelas absorbant en forme de sablier, ayant une forme générale rectangulaire avec deux échancrures latérales opposées, pour une couche-culotte, comprenant, dans le sens de sa longueur, deux zones d'extrémités (5, 6) et une zone intermédiaire d'entrejambe (7) de largeur inférieure à celle des zones d'extrémité, le matelas étant constitué de deux parties de matelas (8, 9) superposées, ayant des formes telles que le matelas présente, dans le sens de la largeur, une partie centrale d'épaisseur supérieure à celle des parties latérales, caractérisé par le fait que le matelas est constitué de deux parties de matelas (8, 9) identiques ayant chacune la même longueur que le matelas et une largeur inférieure à la largeur du matelas dans les zones d'extrémité, l'un des deux bords longitudinaux de chaque partie de matelas étant rectiligne et l'autre muni d'une échancrure (4) identique aux échancrures du matelas, les deux parties de matelas (8, 9) étant superposées partiellement, en position inversée, sous la forme d'un matelas composite de manière que leurs bords rectilignes se chevauchent et que leurs bords munis d'une échancrure soient situés sur les côtés opposés du matelas composite.

2. Matelas suivant la revendication 1, caractérisé par le fait que les deux parties de matelas se chevauchent sur une largeur égale ou inférieure à la largeur de la zone d'entrejambe du matelas composite.

3. Procédé de fabrication en continu de matelas absorbants suivant la revendication 1 ou 2, à partir d'un matériau absorbant en forme de bande, caractérisé par le fait qu'on divise en continu la bande de matériau absorbant dans le sens longitudinal en deux bandes partielles suivant une ligne de découpe ondulée symétrique par rapport à la ligne longitudinale médiane de la bande, la période de la ligne de découpe ondulée correspondant à la longueur d'un matelas absorbant à fabriquer, qu'on décale l'une des bandes partielles ainsi obtenues par rapport à l'autre bande partielle transversalement sur l'autre bande partielle d'une valeur supérieure à la largeur minimale de chaque bande partielle et longitudinalement d'une valeur égale à 1/2, 1 1/2, 2 1/2 ... périodes de la ligne de découpe ondulée, de manière à obtenir une bande composite dont les deux bords latéraux sont ondulés de façon symétrique et dont la partie centrale présente une épaisseur double de celle des parties latérales, et qu'on coupe la bande composite, à des intervalles correspondant à une période de la ligne de découpe ondulée, transversalement dans les zones de largeur maximale de la bande composite.

4. Procédé suivant la revendication 3, caractérisé par le fait qu'on découpe la bande par une ligne de découpe à ondes trapézoïdales formée d'une alternance de segments de droite longitudinaux, de même longueur, situés à égale distance de part et d'autre de la ligne longitudinale médiane de la bande, et de segments obliques, inversés, reliant lesdits segments de droite entre eux.

5. Procédé suivant la revendication 3 ou 4, caractérisé par le fait qu'on décale l'une des bandes partielles transversalement et longitudinalement en lui faisant décrire une boucle autour d'un rouleau dont l'axe est situé dans un plan parallèle au plan de la bande, au-dessus ou en dessous du plan de cette dernière, et est incliné par rapport à la direction transversale de la bande.

6. Procédé suivant l'une quelconque des revendications 3 à 5, caractérisé par le fait qu'on décale l'une des bandes partielles transversalement d'une valeur au moins égale à la largeur maximale de la bande partielle.

## Claims

1. Hourglass-shaped absorbent pad, having a rectangular general shape with two opposite lateral indentations, for a nappy-pant, comprising, in the direction of its length, two end zones (5, 6) and an intermediate crotch zone (7) of width smaller than that of the end zones, the pad consisting of two superimposed pad parts (8, 9), of shapes such that the pad has, in the direction of its width, a central part of thickness greater than that of the lateral parts, characterized in that the pad consists of two identical pad parts (8, 9) each having the same length as the pad and a width smaller than the width of the pad in the end zones, one of the two longitudinal edges of each pad part being rectilinear and the other provided with an indentation (4) identical to the indentations of the pad, the two pad parts (8, 9) being partially superimposed, in the inverse position, in the form of a composite pad so that their rectilinear edges overlap and their edges provided with an indentation are situated on the opposite sides of the composite pad.

2. Pad according to Claim 1, characterized in that the two pad parts overlap over a width equal to or lower than the width of the crotch zone of the composite pad.

3. Process for the continuous manufacture of absorbent pads according to Claim 1 or 2, from an absorbent material in the form of a strip, characterized in that the strip of absorbent material is continuously divided in the longitudinal direction into two partial strips along a wavy cutting line symmetrical relative to the median longitudinal line of the strip, the period of the wavy cutting line corresponding to the length of an absorbent pad to be manufactured, in that one of the partial strips thus obtained is offset relative to the other partial strip in the transverse direction over the other partial strip by a value greater than the minimum width of each partial strip and in the longitudinal direction by a value equal to ½, 1½, 2½ etc periods of the wavy cutting line, so as to obtain a composite strip of which the two lateral edge are wavy in a symmetrical fashion and of which the central part has a thickness twice that of the lateral parts, and in that the composite strip is cut, at intervals corresponding to one period of the wavy cutting line, in the transverse direction in the maximum width zone of the composite strip.

4. Process according to Claim 3, characterized in that the strip is cut by a trapezoidal wave cutting line formed by an alternation of longitudinal straight line segments, of identical length, situated equal distance on either side of the median longitudinal line of the strip, and of inverted, oblique segments joining the said straight line segments to one another.

5. Process according to Claim 3 or 4, characterized in that one of the partial strips is offset in the transverse and longitudinal directions by making it describe a loop around a roller of which the axis is situated in a plane parallel to the plane of the strip, above or below the plane of the latter, and is inclined relative to the transverse direction of the strip.

6. Process according to any one of Claims 3 to 5, characterized in that one of the partial strips is offset in the transverse direction by a value at least equal to the maximum width of the partial strip.

## Patentansprüche

1. Saugfähiges Kissen in Form einer Sanduhr, das eine im wesentlichen rechteckige Form mit zwei seitlichen, gegenüberliegenden Ausschnitten hat, für ein Windelhöschen, und das in Längsrichtung zwei Endbereiche (5, 6) und einen dazwischenliegenden Schrittbereich (7) mit einer Breite aufweist, die kleiner ist als die der Endzonen, wobei das Kissen von zwei übereinanderliegenden Teilkissen (8, 9) gebildet wird, die so gestaltet sind, daß das Kissen in Querrichtung einen mittleren Bereich aufweist, der eine größere Dicke hat als jene der seitlichen Bereiche, dadurch gekennzeichnet, daß das Kissen von zwei gleichen Teilkissen (8, 9) gebildet ist, von denen jedes die gleiche Länge wie das Kissen und eine Breite hat, die kleiner ist als die Breite des Kissens in den Endbereichen, daß einer der Längsränder jedes Teilkissens gerade ist und der andere mit einem Ausschnitt (4) versehen ist, der gleich ist wie der Ausschnitt des Kissens, wobei die beiden Teilkissen (8, 9) in entgegengesetzter Lage zur Form eines zusammengesetzten Kissens derart teilweise übereinanderliegend angeordnet sind, daß ihre geraden Ränder sich überlappen und daß ihre mit einem Ausschnitt versehenen Ränder auf den gegenüberliegenden Seiten des zusammengesetzten Kissens angeordnet sind.

2. Kissen nach Anspruch 1, dadurch gekennzeichnet, daß die zwei Teilkissen sich über eine Breite gleich oder kleiner der Breite des Schrittbereiches des zusammengesetzten Kissens überlappen.

3. Verfahren zur kontinuierlichen Herstellung von saugfähigen Kissen nach Anspruch 1 oder 2, ausgehend von einem bandförmigen saugfähigen Werkstoff, dadurch gekennzeichnet, daß man das Band aus saugfähigem Werkstoff in Längsrichtung längs einer welligen zur Längsmittellinie des Bandes symmetrischen Schnittlinie kontinuierlich in zwei Teilbänder teilt, wobei die Periode der welligen Schnittlinie der Länge eines herzustellenden, saugfähigen Kissens entspricht, daß man eines der so erhaltenen Teilbänder gegenüber dem anderen Teilband in Querrichtung auf das andere Teilband in einem Ausmaß versetzt, das größer ist als die kleinste Breite jedes Teilbandes, und in Längsrichtung in einem Ausmaß gleich 1/2-, 1 1/2-, 2 1/2-Perioden der welligen Schnittlinie versetzt, so daß ein zusammengesetztes Band erhalten wird, dessen beide Seitenränder symmetrisch gewellt sind, und dessen mittlerer Teil doppelt so dick ist wie die Seitenteile, und daß man das zusammengesetzte Band in Abständen entsprechend einer Periode der gewellten Schnittlinie in den Bereichen der größten Breite des zusammengesetzten Bandes quer durchschneidet.

4. Verfahren nach Anspruch 3, dadurch gekennzeichnet, daß man das Band längs einer Schnittlinie mit trapezförmigen Wellen durchschneidet, die von geraden, längslaufenden Abschnitten mit gleicher Länge, die abwechselnd im gleichen Abstand auf beiden Seiten der Längsmittellinie des Bandes angeordnet sind, und von entgegengesetzt schrägen Abschnitten, welche die geraden Abschnitte miteinander verbinden, gebildet ist.

5. Verfahren nach Anspruch 3 oder 4, dadurch gekennzeichnet, daß man eines der Teilbänder in Quer- und in Längsrichtung verschiebt, indem man es eine Schlaufe um eine Rolle beschreiben läßt, deren Achse in einer zur Ebene des Bandes parallelen Ebene oberhalb oder unterhalb der Ebene desselben liegt und zur Querrichtung des Bandes geneigt ist.

6. Verfahren nach einem der Ansprüche 3 bis 5, dadurch gekennzeichnet, daß man das eine der Teilbänder quer in einem Ausmaß versetzt, das wenigstens gleich der maximalen Breite des Teilbandes ist.

## FIG.1

## FIG.2

EP 0 218 857 B1

FIG.3

FIG.4